# EUROPEAN PATENT APPLICATION

(11) **EP 1 800 674 A1**
(43) Date of publication of application: **27.06.2007**
(21) Application number: 06256219.4
(22) Date of filing: 06.12.2006
(51) Int. Cl.: A61K 31/122, A61K 36/05, A61P 3/04, A61P 3/06

(54) **Agent for preventing metabolic syndrome**

(30) Priority: 14.12.2005 JP 2005361038; 17.02.2006 JP 2006041477
(71) Applicant: YAMAHA HATSUDOKI KABUSHIKI KAISHA, Iwata-shi, Shizuoka 438-8501 (JP)
(72) Inventor: Okada, Yumika, Iwata-shi Shizuoka 438-8501 (JP); IIo, Kumiko, Iwata-shi Shizuoka 438-8501 (JP)
(74) Representative: Schlich, George William

(57) **Abstract**

Novel, safe, and highly effective agents for preventing insulin resistance and for preventing metabolic syndrome that contain astaxanthin and/or an ester thereof as an active component are provided. Even when a high-fat diet intake is continued, insulin resistance and subsequent hyperinsulinemia are suppressed by using the agent for preventing insulin resistance and the agent for preventing metabolic syndrome according to the present invention. Furthermore, suppression of fat degradation is thus inhibited and promotion of fat synthesis also is inhibited in adipose tissues, or glucose uptake into adipose tissues is inhibited, so that accumulation of fat within adipocytes is suppressed. Therefore, various diseases or symptoms having a relation to insulin resistance can be prevented or alleviated.

## Description

### BACKGROUND OF THE INVENTION

### Field of the Invention

The present invention relates to an agent for preventing metabolic syndrome. More specifically, the present invention relates to an agent for preventing metabolic syndrome, an agent for preventing insulin resistance, and an agent for preventing a disease having a relation to insulin resistance, the agents containing astaxanthin and/or an ester thereof as an active component.

### Description of the Related Art

Life-style related diseases such as hypertension, hyperlipemia, and diabetes tend to occur concurrently. It has become clear that this is attributable to obesity (visceral fat obesity) in which fat accumulates around the internal organs. A pathological condition induced by visceral fat obesity and characterized by the coincidence of many risk factors causing arteriosclerosis is referred to as metabolic syndrome, and metabolic syndrome is a risk factor for arteriosclerosis as well as cardiovascular and cerebrovascular diseases caused thereby.

At the present time, the diagnostic criteria for metabolic syndrome in Japan are that accumulation of visceral fat is present (waist circumference is 85 cm or more in men or 90 cm or more in women) and that at least two of the following conditions are present: hypertension including borderline hypertension, hyperlipemia or low HDL (high density lipoprotein), and hyperglycemia.

Moreover, according to the U.S. guidelines for treatment of hyperlipemia (ATP III: Adult Treatment Panel III, NCEP National Cholesterol Education Program), metabolic syndrome is diagnosed when three of the following conditions (i) to (v) are present: (i) waist (abdominal circumference) is 102 cm or more in men or 88 cm or more in women; (ii) neutral fat in blood is 150 mg/dl or more; (iii) HDL cholesterol is less than 40 mg/dl in men or less than 50 mg/dl in women; (iv) blood pressure is such that the maximal blood pressure is 130 mmHg or more or the minimal blood pressure is 85 mmHg or more; and (v) fasting blood glucose level is 110 mg/dl or more.

Furthermore, the diagnostic criteria for metabolic syndrome according to the WHO are that hyperinsulinemia is present or fasting blood glucose level is 110 mg/dl or more, plus that two of the following conditions (a) to (d) are present: (a) visceral obesity (waist/hip ratio > 0.9 (men), > 0.85 (women) or BMI of 30 or more or abdominal circumference of 94 cm or more); (b) lipid metabolism abnormality (neutral fat in blood of 150 mg/dl or more or HDL cholesterol of less than 35 mg/dl in men or less than 39 mg/dl in women); (c) hypertension (140/90 mmHg or more or during treatment with an antihypertensive agent); and (d) microalbuminuria (urinary albumin excretion rate of 20 µg/min or more or urinary albumin/creatinine ratio of 30 mg/g creatinine or more).

Obesity is caused when caloric expenditure is lower than caloric intake and the energy source that thus has not been expended accumulates as body fat. The causes of body fat accumulation due to excess energy include lack of exercise, improper eating habit, stress, lipid metabolism abnormality (disorder), excessive secretion of insulin, enlargement of adipocytes, and lack of brown adipocytes. Visceral fat described above is fat found in the mesentery located within the peritoneal cavity, and visceral adipocytes tend to store fat within the individual cells.

When fat accumulates in adipocytes, various types of adipokines (e.g., TNFα, resistin, etc.) are secreted by the adipocytes, causing insulin resistance (i.e., decreased insulin sensitivity). As a result, blood glucose levels can no longer be sufficiently lowered, so that insulin is over-secreted in order to control blood glucose levels, resulting in hyperinsulinemia. When hyperinsulinemia occurs, metabolic syndrome is caused by the action of excess insulin on lipid metabolism and the like.

Insulin is a peptide hormone produced and secreted by β cells in the islets of Langerhans of the pancreas and acts primarily on muscles (the skeletal muscle and the heart muscle), adipose tissues, and the liver. When insulin binds to insulin receptors present on muscle cells and adipocytes, a tyrosine kinase is activated and signals are transmitted into the cells, and thus various activities (physiological effects) are produced.

In adipose tissues, excess insulin suppresses degradation of fat or promotes synthesis of fat, so that additional fat accumulates. Alternatively, insulin promotes glucose uptake into adipocytes, so that fat accumulates within the adipocytes via the glycolytic pathway. Thus, due to excessive secretion of insulin, adipocytes store additional fat and hypertrophy. As described above, there is a very close relation between insulin and lipid metabolism.

When insulin resistance occurs and therefore hyperinsulinemia occurs, fat synthesis is promoted, as described above. Moreover, the enzyme activity of lipoprotein lipase (LPL) is decreased, the metabolism (catabolism) of chylomicron and very low density lipoprotein (VLDL) is impaired, and furthermore, triglyceride production (VLDL production) in the liver is increased. Accordingly, neutral fat in blood is increased, resulting in hypertriglyceridemia or hyper-VLDL-lipoproteinemia (type IV hyperlipemia). At this time, hyper-VLDL-lipoproteinemia involves an increase in the production of LDL, which is produced by the catabolism of VLDL, and the activity of LDL receptors is decreased, resulting in hyper-LDL-lipoproteinemia (hypercholesterolemia). Furthermore, hyper-VLDL-lipoproteinemia involves an increase in the metabolism of high density lipoprotein (HDL) and also a decrease in the production of HDL, resulting in hypo-HDL-lipoproteinemia. These various types of hyperlipemia cause arteriosclerosis, which can lead to cerebral thrombosis, myocardial infarction, and other diseases.

When hyperinsulinemia occurs, non insulin-dependent (type II) diabetes mellitus develops in individuals with a predisposition to diabetes, and as the disease progresses further, pancreatic β cells become exhausted and insulin-dependent diabetes mellitus develops. Diabetes induces various complications such as retinopathy, neuropathy, nephropathy, and cardiovascular disorders. Moreover, once diabetes develops, it is difficult to recover completely, so that prevention of diabetes is very important.

Furthermore, when hyperinsulinemia occurs, insulin increases the activities of sympathetic nerves and the renin-angiotensin system, resulting in, for example, vascular endothelial dysfunction, and thus, blood pressure is increased, which leads to hypertension and further to arteriosclerosis. In this manner, in metabolic syndrome, hypertrophy of visceral fat induces insulin resistance and hyperinsulinemia, and consequently, hyperlipemia, diabetes, and hypertension occur as complications, resulting in an increased risk of developing arteriosclerosis.

Carotenoids are naturally-occurring substances having an antioxidative effect, and their various bioactivities have attracted interest. However, few studies have been conducted to investigate the action of carotenoids on obesity and adipocytes or insulin resistance. It has been reported only that a carotenoid derived from a vegetable or a fruit suppresses the differentiation induced by insulin of preadipocytes into adipocytes (Japanese Laid-Open Patent Publication No. 2003-95930). However, obesity, as discussed above, is caused not only by an increase in the number of adipocytes but also, especially in visceral fat obesity, by accumulation of fat within adipocytes. Moreover, it has been reported also that differentiation of preadipocytes into adipocytes is accompanied by induction of expression of adiponectin, but when differentiation is impaired and thus fat atrophies, adiponectin becomes deficient, causing a metabolic disorder, which leads to metabolic syndrome (Takashi Kadowaki et al., "The Role of Adiponectin in Molecular Mechanisms of Diabetes and Cardiovascular Diseases", proceedings of The 128th Japanese Association of Medical Sciences Symposium on "Diabetes Mellitus and Atherosclerosis", December 2, 2004, pp. 34-45). Therefore, since carotenoids merely suppress differentiation into adipocytes, it is doubtful whether carotenoids have a sure anti-obesity action.

### SUMMARY OF THE INVENTION

It is an object of the present invention to provide safe and highly effective agents for preventing insulin resistance and for preventing metabolic syndrome.

The present invention provides an agent for preventing insulin resistance, the agent comprising astaxanthin and/or an ester thereof as an active component.

In one embodiment, the insulin resistance is caused by obesity.

The present invention also provides an agent for preventing a disease having a relation to insulin resistance, the agent comprising astaxanthin and/or an ester thereof as an active component.

The present invention further provides an agent for preventing metabolic syndrome, the agent comprising astaxanthin and/or an ester thereof as an active component.

In an embodiment, the astaxanthin and/or the ester thereof is derived from a microalga belonging to the genus *Haematococcus.*

In addition, the present invention provides a use of astaxanthin and/or an ester thereof in the manufacture of an agent for preventing insulin resistance.

In an embodiment, the insulin resistance is caused by obesity.

In one embodiment, the agent is for use in obese patients.

The present invention further provides a use of astaxanthin and/or an ester thereof in the manufacture of an agent for preventing a disease having a relation to insulin resistance.

The present invention also provides a use of astaxanthin and/or an ester thereof in the manufacture of an agent for preventing metabolic syndrome.

In addition to the above, the invention provides pharmaceutical compositions and health food supplements comprising astaxanthin and/or an ester thereof.

The present invention also provides a method of manufacturing an agent according to the invention, the method comprising (a) growing in culture a microalga belonging to the genus *Haematococcus*; and (b) extracting and purifying astaxanthin and/or an ester thereof from the culture.

According to the present invention, novel and highly effective agents for preventing and/or treating insulin resistance, for preventing and/or treating a disease having a relation to insulin resistance, and for preventing and/or treating metabolic syndrome are provided. Even when a high-fat diet intake is continued, lipid metabolism abnormality is reduced by using the agent for preventing insulin resistance or the agent for preventing metabolic syndrome according to the present invention. More specifically, since, in adipose tissues, suppression of fat degradation is inhibited or promotion of fat synthesis also is inhibited, accumulation of fat is suppressed. Moreover, an increase in the blood glucose level is suppressed and excessive secretion of insulin is suppressed, so that glucose uptake into adipose tissues is suppressed, and thus accumulation of fat within adipocytes is suppressed. Therefore, obesity is suppressed, and furthermore, insulin resistance caused by obesity and subsequent hyperinsulinemia and metabolic syndrome can be prevented or suppressed. Accordingly, life-style related diseases such as hypertension, hyperlipemia, and diabetes can be prevented. Furthermore, the agent for preventing insulin resistance, the agent for preventing a disease having a relation to insulin resistance, or the agent for preventing metabolic syndrome according to the present invention has very low toxicity and thus offers a high degree of safety.

### BRIEF DESCRIPTION OF THE DRAWINGS

Fig. 1 is a graph showing the change over time in body weight of mice during a period of 16 weeks from the start of a test.
Fig. 2 is a graph showing the weights of subcutaneous fat and visceral fat of the mice in each group at the end of the test.
Fig. 3 is a graph showing the proportions of the weights of various adipose tissues and the liver to body weight of the mice in each group at the end of the test.
Fig. 4 is a graph showing the insulin concentration in blood of the mice in each group at the end of the test.
Fig. 5 is a graph showing the blood glucose level of the mice in each group at the end of the test.
Fig. 6 is a graph showing for each subject the change in the concentration of neutral fat in blood before and after ingestion of astaxanthin capsules.
Fig. 7 is a graph of the average values of the concentrations of neutral fat in blood of the subjects before and after the ingestion of the astaxanthin capsules.
Fig. 8 is a graph showing for each subject the change in the diastolic blood pressure before and after the ingestion of the astaxanthin capsules.

### DESCRIPTION OF THE PREFERRED EMBODIMENTS

Astaxanthin or an ester thereof used in the present invention is a carotenoid represented by the following formula: 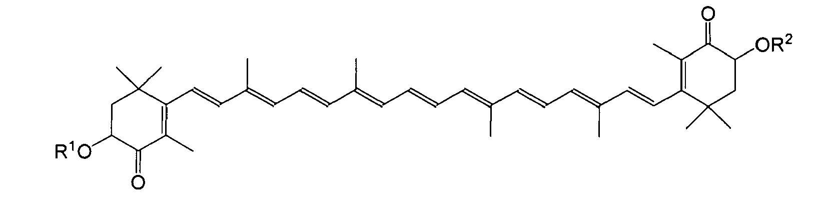 wherein R¹ and R² are both hydrogen in the case of astaxanthin, and R¹ and R² are each independently a hydrogen atom or a fatty acid residue provided that at least one of R¹ and R² is a fatty acid residue in the case of an ester of astaxanthin. Examples of the fatty acid residue in the ester of astaxanthin include, but are not limited to, saturated fatty acids such as palmitic acid and stearic acid or unsaturated fatty acids such as oleic acid, linoleic acid, a-linolenic acid, γ-linolenic acid, bishomo-γ-linolenic acid, arachidonic acid, eicosapentaenoic acid, and docosahexaenoic acid, or any combination thereof. The astaxanthin ester of the present invention can be any mono- or diester, homogeneous or non-homogeneous. Astaxanthin has a structure in which an additional oxo group and an additional hydroxy group are present at each end of a β-carotene molecule, so that unlike for β-carotene, the stability of the molecule is low. On the other hand, an ester form (e.g., as obtained in an extract from krill) in which the hydroxy groups at both ends are esterified with an unsaturated fatty acid is more stable.

Astaxanthin or an ester thereof used in the present invention may be chemically synthesized or derived from a naturally-occurring product. Examples of the naturally-occurring products in the latter case include red yeast; the shell of crustaceans such as Tigriopus (red water flea) and krills; and microalgae such as green algae, which contain astaxanthin and/or an ester thereof. In the present invention, any extract containing astaxanthin and/or esters thereof produced by any method can be used. Generally, extracts from those naturally-occurring products can be used, and the extracts may be crude or purified if necessary. In the present invention, a crude extract or a crushed powder of naturally-occurring products, or a purified product or a chemically synthesized product, if necessary, that contains such astaxanthin and/or esters thereof can be used either alone or in combination. In view of the chemical stability, an ester form of astaxanthin is preferably used.

The agent for preventing insulin resistance according to the present invention can prevent or suppress insulin resistance. That is to say, the agent can reduce obesity, which is a factor inducing insulin resistance. More specifically, the agent prevents induction of insulin resistance and inhibits suppression of fat degradation or inhibits also promotion of fat synthesis in adipose tissues, thereby suppressing accumulation of fat. Moreover, since an increase in the blood glucose level is suppressed and excessive secretion of insulin is suppressed, glucose uptake into adipose tissues also can be suppressed. Furthermore, the concentration of neutral fat in blood also can be controlled. Therefore, the agent is useful also as an agent for preventing hyperinsulinemia induced by insulin resistance and preventing subsequent metabolic syndrome. In this specification, preventing metabolic syndrome refers to preventing or alleviating a state in which at least one of the conditions listed above as the diagnostic criteria is present. Moreover, the agent for preventing insulin resistance according to the present invention is useful also as an agent for preventing a disease having a relation to insulin resistance. Examples of the disease having a relation to insulin resistance include various life-style related diseases, such as hyperlipemia, arteriosclerosis, hypertension, myocardial infarction, cerebrovascular disorders, cerebral infarction, angina pectoris, pancreatitis, diabetes, fatty liver, metabolic disorders, and obesity, and other diseases.

The route of administration of the agent for preventing insulin resistance, the agent for preventing a disease having a relation to insulin resistance, or the agent for preventing metabolic syndrome according to the present invention may be either oral or parenteral. The dosage form is selected appropriately according to the route of administration. Examples thereof include parenteral solutions, infusion solutions, powders, granules, tablets, capsules, pills, enteric-coated preparations, troches, liquids for internal use, suspensions, emulsions, syrups, liquids for external use, poultices, nose drops, ear drops, eye drops, inhalants, ointments, lotions, suppositories, and enteral nutrients. These can be used either alone or in combination depending on the condition of a disease. To prepare these dosage forms, auxiliary substances commonly used in the field of pharmaceutical manufacturing technology, such as excipients, binders, antiseptics, antioxidants, disintegrators, lubricants, and flavoring agents, can be used as necessary.

The dose of the agent for preventing insulin resistance, the agent for preventing a disease having a relation to insulin resistance, or the agent for preventing metabolic syndrome according to the present invention varies depending on the purpose of administration, the individual to be administered (sex, age, body weight, etc.), and the severity and nature of the disease, and can be determined by a person skilled in the art. Usually, the dose for an adult in terms of free or unesterified form of astaxanthin may be 0.1 mg to 2 g, preferably 1 mg to 1 g, more preferably 4 mg to 500 mg (e.g. 4 mg) per day in the case of oral administration, while it may be 0.01 mg to 1 g, preferably 0.015 mg to 750 mg, more preferably 0.1 mg to 500 mg (e.g. 4 mg) per day in the case of parenteral administration.

The agent for preventing insulin resistance, the agent for preventing a disease having a relation to insulin resistance, or the agent for preventing metabolic syndrome according to the present invention can be used not only as pharmaceuticals as described above, but also as the category of products regulated as "quasi-drugs", cosmetics, food products, nutritional supplements, foods and drinks, and other similar products. When used as quasi-drugs or cosmetics, the agent may be used in conjunction with various auxiliary substances commonly used in the field of quasi-drugs or cosmetics, or other technologies, if necessary. Alternatively, when used as food products, nutritional supplements, or foods and drinks, the agent may be used in conjunction with additives commonly used for food products, for example, sweeteners, spices, seasonings, antiseptics, preservatives, germicides, and antioxidants, if necessary. The agent may be used in a desired form such as solution, suspension, syrup, granule, cream, paste, or jelly, or may be shaped, if necessary. The ratio of the agent contained in these products is not particularly limited, and can be selected appropriately according to the intended purpose, the mode of usage, and the amount of usage.

### Examples

### Preparation Example 1: Preparation of Astaxanthin Monoester

An astaxanthin monoester was prepared in the following manner. *Haematococcus pluvialis* K0084 strain was cultivated at 25°C under irradiation with light while bubbling a gas containing 3% CO₂ into the medium and under nutrient stress condition (i.e. nitrogen source deprivation), and then was encysted. The encysted cells were disrupted by means commonly used by those skilled in the art, and a lipophilic fraction was extracted with ethanol. The extract contained lipids such as triglyceride in addition to astaxanthins. The extract was subjected to column chromatography using a synthetic resin adsorbent to give a purified product containing astaxanthin monoesters. This purified product was analyzed by HPLC, and it was confirmed that this purified product contained an astaxanthin monoester having a molecular weight of 858 as the main component, did not contain the free form of astaxanthin, the diester form of astaxanthin, and triglyceride, and that it contained a small amount of diglyceride.

### Preparation Example 2: Preparation of Astaxanthin Capsule

Astaxanthin was prepared in the following manner. *Haematococcus pluvialis* K0084 strain was cultivated at 25°C under irradiation with light while bubbling a gas containing 3% CO₂ into the medium and under nutrient stress condition (i.e. nitrogen source deprivation), and then was encysted. The encysted cells were disrupted by means commonly used by those skilled in the art, and a lipophilic fraction containing astaxanthin was extracted with ethanol. The extract was concentrated under reduced pressure, and the ethanol was evaporated to give an extract containing astaxanthin in an amount of 9.9% expressed in terms weight of the free form.

Soft capsules containing the components shown in Table 1 below per capsule were prepared using the extract containing astaxanthin in an amount of 9.9% expressed in terms weight of the free form.

**Table 1**

| Component | Weight | Note |
|---|---|---|
| Haematococcus extract | 40 mg | Astaxanthin 9.9 wt% |
| Glycerin fatty acid ester (Nikko Chemicals Co., Ltd.) | 20 mg | as emulsifier |
| Safflower oil (The Nisshin OilliO Group. Ltd.) | 190 mg | |
| Total | 250 mg | |

The obtained soft capsules contained astaxanthin in an amount of 4 mg per capsule expressed in terms of weight of the free form.

### Example 1: Effect of astaxanthin monoester on obese model mice fed with a high-fat diet

Astaxanthin was administered to obese model mice fed with a high-fat diet, and the change in body weight, the amount of subcutaneous fat (in the inguinal region and the back), the amount of visceral fat (around the reproductive organs and around the kidney), the liver weight, the blood glucose level, and the insulin concentration in blood were examined in the following manner.

Four week old male C57BL/6J strain mice (SPF) purchased from CHARLES RIVER LABORATORIES JAPAN, INC. were used. The mice were preliminarily fed for 8 days and used for the test after they reached the age of 5 weeks. The mice were divided into three groups of 8 each, that is, a normal diet group, a high-fat diet group, and a high-fat diet + astaxanthin (AX) group, so that the average body weight was equal among the groups.

During the preliminary feeding period, the mice were given an ordinary powder diet (MF, Oriental Yeast Co., Ltd.), and during the test period of 16 weeks, the mice were given the ordinary powder diet or a high-fat diet having the composition shown in Table 2 below. As to drinking water, the mice were allowed to drink freely sterile distilled water from a water supply bottle.

**Table 2**

| Component | Composition of high-fat diet (part by weight) |
|---|---|
| Beef tallow | 400 |
| Corn starch | 100 |
| Glucose | 90 |
| AIN- 76TM mineral mix | 40 |
| AIN- 76TM vitamin mix | 10 |
| Casein | 360 |

| | |
|---|---|
| AIN- 76 compositions from Oriental Yeast Co., Ltd. | |

The astaxanthin monoester prepared in Preparation Example 1 was dissolved in an olive oil (Wako Pure Chemical Industries, Ltd.) to prepare a solution containing astaxanthin monoester at a concentration of 60 mg/mL. This solution was administered to the high-fat diet + AX group and the olive oil to the other two groups in a volume of 0.05 mL/10 g body weight every day for 16 weeks from the start of the test (at the age of 5 weeks) to the age of 21 weeks, using a sonde for oral administration in the mice.

During the test period, the body weight was measured once a week using a scale. At the end of the test, the body weight was measured, and thereafter, the mice were fasted overnight and the blood glucose level was measured in the tail of each mouse using OneTouch Ultra^{™} (manufactured by Life Scan Inc., a blood glucose level measuring device). Next, blood was collected from the heart using a heparinized syringe with heparin Na. Immediately after the collection of blood, blood plasma was separated by centrifugation, and the obtained blood plasma was cryopreserved at -80°C. Then, the liver, the adipose tissue in the inguinal region, the adipose tissue around the reproductive organs, the adipose tissue around the kidney, and the adipose tissue in the back were collected and the weights thereof were measured. Thereafter, the insulin concentration in blood was measured with a commercially available kit (Mouse Insulin ELISA KIT (TMB) (AKRIN-011T): Shibayagi Co., Ltd.), using the blood plasma collected at the end of the test.

The different types of data obtained were expressed as average values ± standard errors for each group. In order to test for statistically significant differences between the high-fat diet group and the high-fat diet + AX group or the normal diet group, a multiple comparison test (ANOVA) was performed using an analysis software (Stat View, Abacus Inc., USA), and a comparison between the groups was performed using Fisher's PLSD multiple comparison test. Differences were considered statistically significant when p < 0.05.

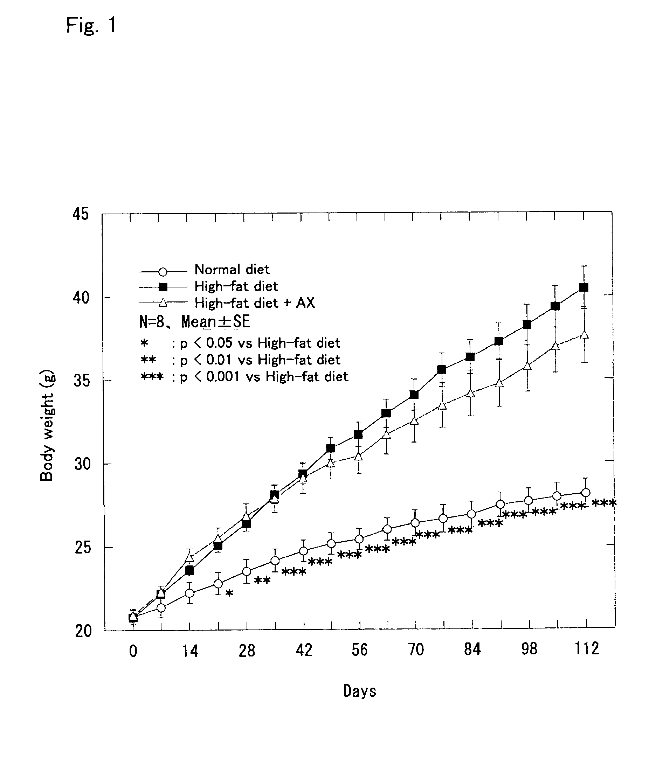

Fig. 1 shows the change over time in mean body weight of the mice during the period of 16 weeks from the start of the test. Body weight in the high-fat diet group significantly increased when compared to the normal diet group. Body weight in the high-fat diet + AX group increased more greatly than the normal diet group, but the increase in body weight tended to be suppressed more than in the high-fat diet group.

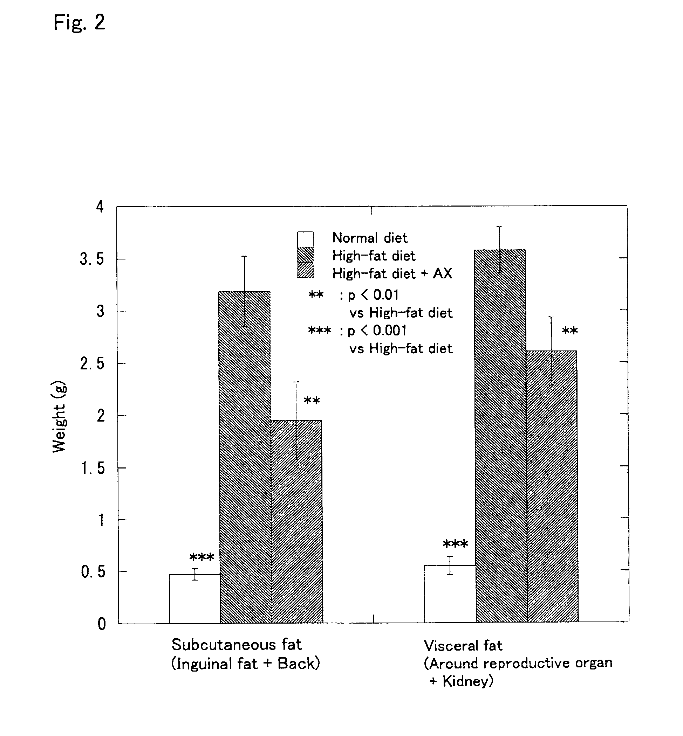

Fig. 2 shows the mean weights of subcutaneous fat and visceral fat of the mice in each group. It can be seen from Fig. 2 that although the amount of fat accumulation both in subcutaneous fat and in visceral fat was considerably increased by intake of the high-fat diet, the accumulation of fat was significantly suppressed when taking astaxanthin together with the high-fat diet.

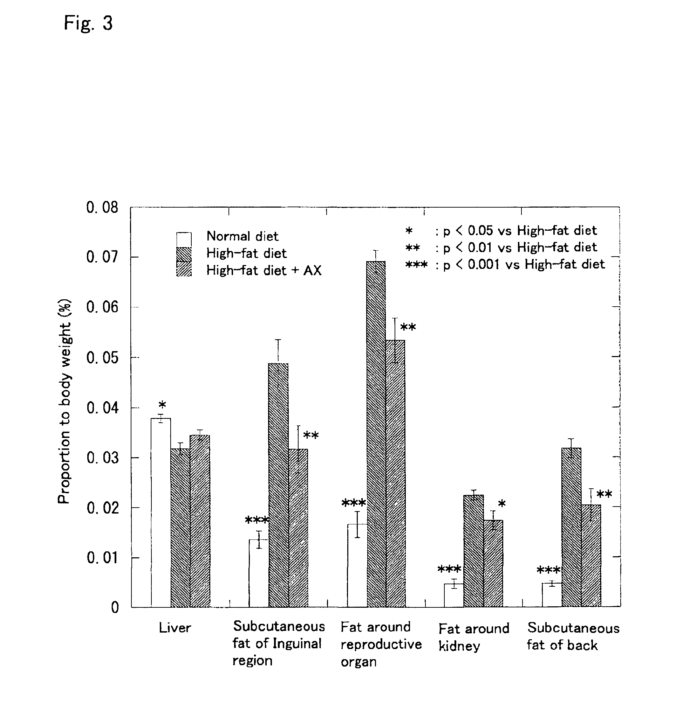

Fig. 3 shows the proportions of the mean weights of the adipose tissues and the liver to mean body weight. The proportions of all of the adipose tissues to body weight were dramatically increased by intake of the high-fat diet, but the increase was significantly suppressed when taking astaxanthin together with the high-fat diet. Moreover, it was found that although the proportion of the liver weight to body weight was reduced by intake of the high-fat diet, the proportion approached that in the case of the normal diet when taking astaxanthin together with the high-fat diet.

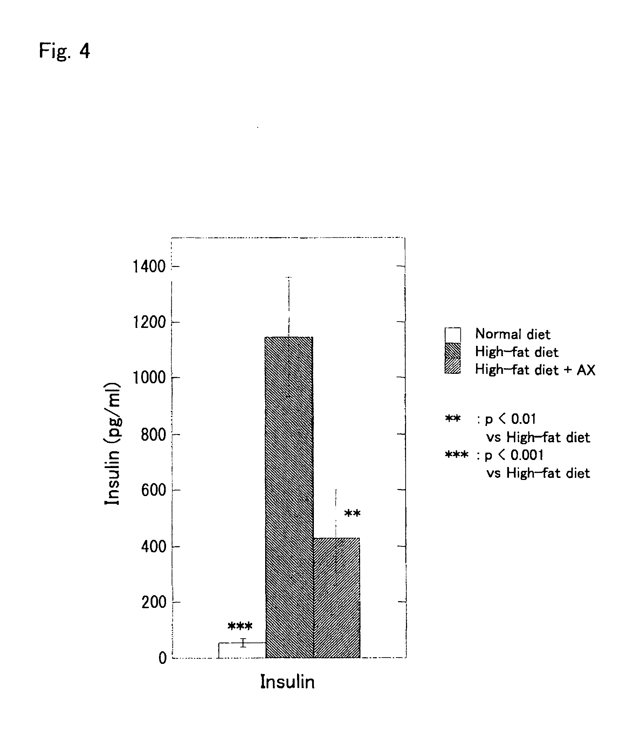

Fig. 4 shows the insulin concentration in blood of the mice in each group. In the high-fat diet group, the insulin concentration was considerably increased. When adipokines are secreted by adipocytes that store fat, insulin sensitivity is reduced (insulin resistance develops), so that blood glucose levels can no longer be lowered sufficiently. It can be considered that insulin was therefore secreted excessively in order to control blood glucose levels. Moreover, the results indicated that since insulin has the action of suppressing fat degradation in adipose tissues, of promoting fat synthesis, and of promoting glucose uptake into adipose tissues, accumulation of fat is promoted by excessive secretion of insulin, resulting in additional fat accumulation (see Figs. 3 and 4). On the other hand, in the high-fat diet + AX group, the insulin concentration was considerably suppressed when compared to the high-fat diet group, and thus, it can be considered that an increase in fat in each adipose tissue was suppressed.

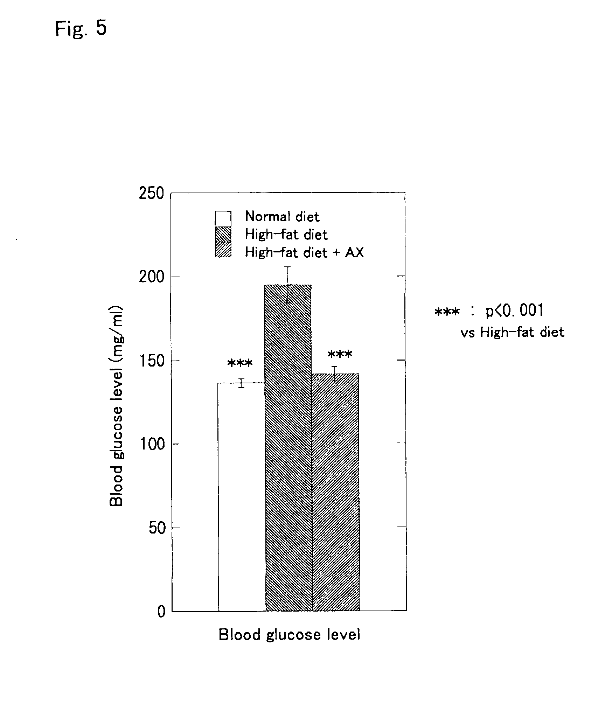

Fig. 5 shows the blood glucose level of the mice in each group. Although the blood glucose level was increased by intake of the high-fat diet, the blood glucose level was maintained at the same level as that of the normal diet group when taking astaxanthin with the high-fat diet. That is to say, the increase in the blood glucose level due to intake of the high-fat diet was significantly suppressed to the level of the normal diet group. This is in good accordance with the results for the insulin concentration in blood described above. Furthermore, the high-fat diet group exhibited insulin resistance in which blood glucose levels are not lowered sufficiently even when insulin is secreted excessively, whereas the astaxanthin administered group showed that blood glucose levels were lowered sufficiently with secretion of a relatively small amount of insulin. This indicates that insulin resistance was prevented. Moreover, it also indicates that diabetes could be prevented, because the increase in the blood glucose level was suppressed.

### Example 2: Effect of astaxanthin on concentration of neutral fat in blood

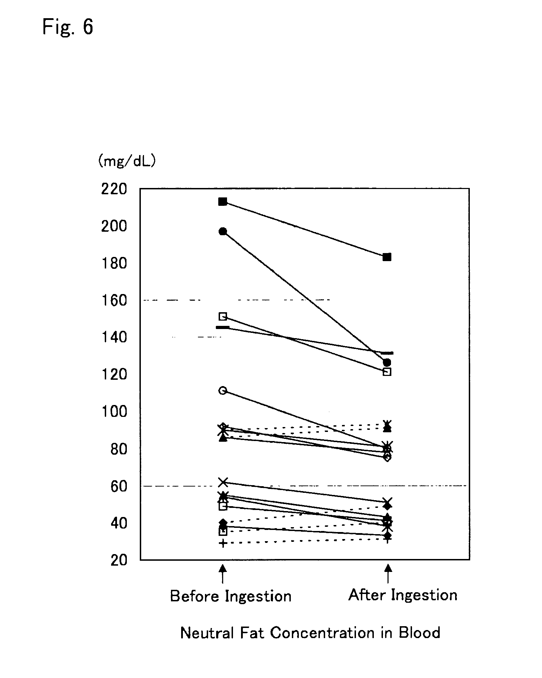

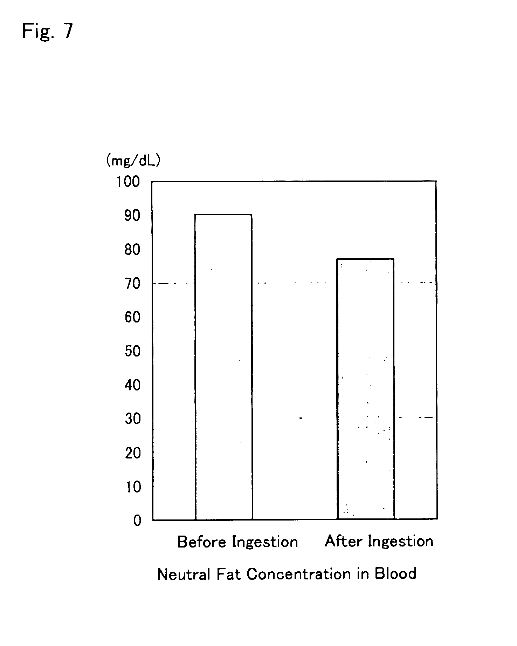

Eighteen subjects consisting of men and women in their twenties to seventies ingested one of the astaxanthin capsules obtained in Preparation Example 2 once a day for four weeks. Blood was collected from each subject before breakfast on the day when the ingestion started, and before breakfast on the day following the day when the ingestion ended, to determine the neutral fat concentration in blood by an enzymatic method using L-type TG-M (Wako Pure Chemical Industries, Ltd.). In the enzymatic method used for this determination, triglyceride (neutral fat) is hydrolyzed by lipoprotein lipase to glycerol. Then, the produced glycerol is further subjected to a colorimetric assay in which an oxidative color-developing agent commonly used in the art is employed in the presence of glycerol kinase and glycerol-3-phosphate oxidase. The results are shown in Figs. 6 and 7. Fig. 6 shows for each subject the change in the concentration of neutral fat in blood before and after the ingestion of the astaxanthin capsules, and Fig. 7 shows the average values of the neutral fat concentrations in blood of all the subjects. In both of the graphs, the values on the vertical axis are in mg/dL.

As can be seen from Fig. 6, the concentration of neutral fat in blood was decreased in thirteen subjects (shown by solid lines) out of the eighteen subjects. Moreover, as shown in Fig. 7, the average value of the neutral fat concentrations of all of the subjects was lowered by 14.7% when compared to the average value before the ingestion of the astaxanthin capsules. In particular, in all of the subjects having neutral fat concentrations of 100 mg/dL or more before the ingestion of the astaxanthin capsules, the neutral fat concentrations were lowered (see Fig. 6), and the average drop was 35.2 mg/dL (21.5% when the value before the ingestion was taken as 100%). Thus, it can be seen that a high neutral fat lowering effect was provided especially in the subjects having high neutral fat concentrations. Generally, normal values of the neutral fat concentration are 35 to 149 mg/dL. After the ingestion of the astaxanthin capsules, the neutral fat concentration was reliably lowered in the subjects having high neutral fat concentrations, whereas the neutral fat concentration was not lowered very much in the subjects having low neutral fat concentrations. From these results, it was found that astaxanthin controls the neutral fat concentration to bring this concentration within a normal value range, without lowering excessively the neutral fat concentration, and thus it is also found that astaxanthin works safely for controlling neutral fat concentration in blood. Moreover, the values for neutral fat concentration of almost all of the subjects were within the normal range after the ingestion. This finding showed that astaxanthin controls the concentration of neutral fat in blood so that it is brought within a normal range.

### Example 3: Effect on blood pressure

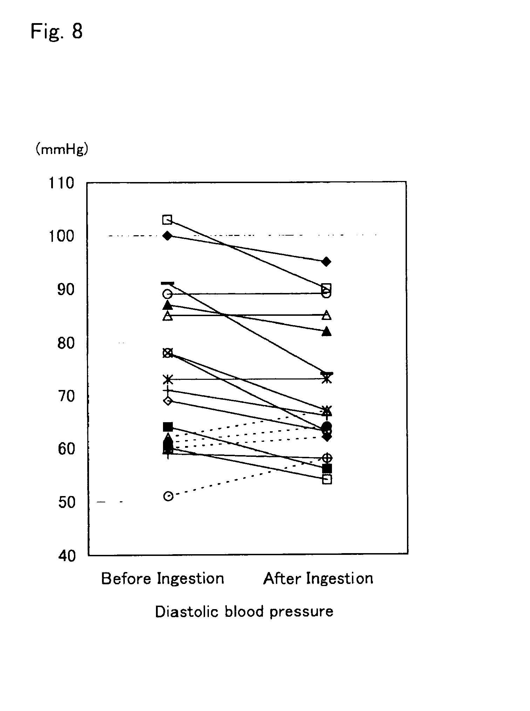

Eighteen subjects consisting of men and women in their twenties to seventies ingested one of the astaxanthin capsules obtained in Preparation Example 2 once a day for four weeks. Diastolic blood pressure of each subject was measured before breakfast on the day when the ingestion started, and before breakfast on the day following the day when the ingestion ended. Fig. 8 shows for each subject the change in the diastolic blood pressure (mmHg) before and after the ingestion of the astaxanthin capsules. In Fig. 8, solid lines indicate the blood pressures of the subjects whose blood pressure was lowered or not changed, and dotted lines indicate that of the subjects whose blood pressure was increased.

As can be seen from Fig. 8, in all of the subjects having diastolic blood pressures of 90 mmHg or more before the ingestion of the astaxanthin capsules, the blood pressure was lowered after the ingestion of the astaxanthin capsules. On the contrary, in the subject having a blood pressure as low as 51 mmHg, the blood pressure was increased to near a normal range (60 to 89 mmHg). Thus, after the ingestion of the astaxanthin capsules, the blood pressure was reliably lowered in the subjects having high blood pressures, whereas the blood pressure was not lowered very much in the subjects having low blood pressures. From these results, it was found that astaxanthin controls the blood pressure to bring it within a normal value range, without lowering excessively the blood pressure, and thus it was also found that astaxanthin works safely for controlling the blood pressure. Moreover, the values for blood pressure of almost all of the subjects were around the normal range after the ingestion. This finding showed that astaxanthin also controls the blood pressure so that it is brought to a normal range, just as is the case with the concentration of neutral fat in blood in Example 2.

### Reference Example 1: Measurement of 50% Lethal Concentration for HUVEC

Human umbilical vein endothelial cells (HUVECs) (ATCC CRL-1730) were obtained from American Type Culture Collection and precultivated in an Endothelial Cell Growth Medium (CELL APPLICATIONS, USA) containing 10% bovine fetal serum supplemented with 1% Antibiotic-Antimycotic (GIBCO BRL, USA) under a 5% CO₂ atmosphere at 37°C.

A Matrigel matrix (BD Biosciences, USA) was melted and kept at 4°C on ice, and then, 50 µL of the matrix were transferred to each well of a 96-well tissue culture plate. The plate was incubated at 37°C for at least one hour to solidify the matrix solution.

On the other hand, the astaxanthin monoester obtained in Preparation Example 1 was dissolved in dimethylsulfoxide (DMSO), and then diluted with distilled water to prepare stock test solutions in which the astaxanthin monoester was contained in 40 (v/v)% DMSO at 25000, 2500, 250, 25, and 2.5 µM, respectively.

Next, 100 µL of a HUVEC suspension (about 2.5 × 10³ cells/well) were poured into the 96-well Matrigel plate under a 5% CO₂ atmosphere at 37°C. After 24 hours, 100 µL of a growth medium and 2 µL of each of the stock test solutions or the vehicle (40 (v/v)% DMSO) were added to two wells each, and incubated for an additional 72 hours. The final concentrations of the astaxanthin monoester were 250, 25, 2.5, 0.25, and 0.025 µM.

After the incubation, 20 µL of a 90% alamarBlue reagent were added to individual wells, and incubated for an additional 6 hours. Then, the fluorescence intensity of each well was measured at an excitation wavelength of 530 nm and an emission wavelength of 590 nm using a Spectrafluor Plus plate reader to count the number of living cells. This measurement is based on the ability of a living cell to change alamarBlue from the non-fluorescent, oxidized form (blue) to the fluorescent, reduced form (red). The 50% lethal concentration was calculated as the concentration at which the number of living cells was 50% of the number of cells at the start of the experiment.

The result indicates that the 50% lethal concentration (LC₅₀) of the astaxanthin monoester for the HUVECs was 250 µM (maximum concentration of the astaxanthin monoester dissolved in DMSO) or more, and thus it was found that the toxicity of the astaxanthin monoester is low.

According to the present invention, novel and highly effective agents for preventing insulin resistance, for preventing a disease having a relation to insulin resistance, and for preventing metabolic syndrome are provided. Even when a high-fat diet intake is continued, lipid metabolism abnormality is reduced by using the agent for preventing insulin resistance or the agent for preventing metabolic syndrome according to the present invention. More specifically, since, in adipose tissues, suppression of fat degradation is inhibited or promotion of fat synthesis is also inhibited, accumulation of fat is suppressed. Alternatively, since an increase in the blood glucose level is suppressed and excessive secretion of insulin is suppressed, glucose uptake into adipose tissues is suppressed, so that accumulation of fat within adipocytes is suppressed. Therefore, obesity is suppressed, and furthermore, insulin resistance and hyperinsulinemia caused by obesity, and metabolic syndrome can be prevented or suppressed. Accordingly, life-style related diseases such as hypertension, hyperlipemia, and diabetes can be prevented.

Astaxanthin and/or an ester thereof, which is an active component in the agent for preventing insulin resistance, the agent for preventing a disease having a relation to insulin resistance, or the agent for preventing metabolic syndrome according to the present invention, has been consumed in food for a long time and has very low toxicity, therefore, astaxanthin and/or an ester thereof has a very high degree of safety. Accordingly, these agents are not only used as pharmaceuticals, but can be used also prophylactically on a daily basis as health food products.

The invention may be embodied in other forms without departing from the spirit or essential characteristics thereof. The embodiments disclosed in this application are to be considered in all respects as illustrative and not limiting. The scope of the invention is indicated by the appended claims rather than by the foregoing description, and all changes which come within the meaning and range of equivalency of the claims are intended to be embraced therein.

## Claims

**1.** An agent for preventing and/or treating insulin resistance, e.g. in a human, comprising astaxanthin and/or an ester thereof as an active component.

**2.** The agent for preventing and/or treating insulin resistance of claim 1, wherein the astaxanthin and/or the ester thereof is derived from a microalga belonging to the genus *Haematococcus.*

**3.** The agent for preventing and/or treating insulin resistance of claim 1 or 2, wherein the insulin resistance is caused by obesity.

**4.** An agent for preventing and/or treating a disease having a relation to insulin resistance, e.g. in a human, comprising astaxanthin and/or an ester thereof as an active component.

**5.** An agent for preventing and/or treating metabolic syndrome, e.g. in a human, comprising astaxanthin and/or an ester thereof as an active component.

**6.** The agent for preventing and/or treating metabolic syndrome of claim 5, wherein the astaxanthin and/or the ester thereof is derived from a microalga belonging to the genus *Haematococcus.*

**7.** Use of astaxanthin and/or an ester thereof in the manufacture of an agent for preventing and/or treating insulin resistance, e.g. in a human.

**8.** The use according to claim 7, wherein the insulin resistance is caused by obesity.

**9.** The use according to claim 8, wherein the agent is for use in obese patients.

**10.** Use of astaxanthin and/or an ester thereof in the manufacture of an agent for preventing and/or treating a disease having a relation to insulin resistance, e.g. in a human.

**11.** The use according to claim 10, wherein the disease is selected from the group consisting of hyperlipemia, arteriosclerosis, hypertension, myocardial infarction, angina pectoris, cerebrovascular disorders, cerebral infarction, pancreatitis, diabetes, fatty liver, metabolic disorders, and obesity.

**11.** Use of astaxanthin and/or an ester thereof in the manufacture of an agent for preventing and/or treating metabolic syndrome, e.g. in a human.

**12.** The agent of any one of claims 1 to 6, or the use according to any one of claims 7 to 11, wherein the agent comprises an ester of astaxanthin, e.g. a monoester.

**13.** A pharmaceutical composition comprising astaxanthin and/or an ester thereof as an active component.

**14.** A health food supplement comprising astaxanthin and/or an ester thereof as an active component.

**15.** A method of manufacturing an agent of any one of claims 1 to 6, comprising:
(a) growing in culture a microalga belonging to the genus *Haematococcus*; and
(b) extracting and purifying astaxanthin and/or an ester thereof from the culture.
